# EUROPEAN PATENT SPECIFICATION

(11) **EP 2 300 090 B1**
(45) Date of publication and mention of the grant of the patent: **18.12.2019**
(21) Application number: 09766114.4
(22) Date of filing: 16.06.2009
(51) Int. Cl.: A61M 25/00, A61M 25/01, A61M 25/06

(54) **DEPLOYING LININGS IN BODY CAVITIES**
AUSKLEIDUNG VON KÖRPERHÖHLUNGEN
DÉPLOIEMENT DE DOUBLURES PROTECTRICES DANS DES CAVITÉS CORPORELLES

(30) Priority: 16.06.2008 GB 0811089
(43) Date of publication of application: 30.03.2011
(73) Proprietor: Scopeguard Limited, Liphook, Hampshire GU30 7U (GB)
(72) Inventor: YOUNG, Antony, John, Southend Essex SS1 3HB (GB); POOLE, George, Anthony, Hampshire SO41 5TB (GB)
(74) Representative: Foster, Mark Charles
(86) International application number: PCT/GB2009/001502
(87) International publication number: WO 2009/153553

(56) References cited:
- EP-A- 0 247 559
- WO-A-00/32118
- WO-A-03/084584
- US-A1- 2001 044 595

## Description

### Field of the Disclosure

The present disclosure relates to a method of lining a body cavity and to a lining device for lining a body cavity.

### Background of the Disclosure

Around one in four hospital patients have an indwelling urinary catheter, approximately 5% of these go on to develop a urinary tract infection, some of these are associated with bacteriemia. Infections associated with catheters are one of the leading causes of hospital acquired infections. They also cause considerable morbidity in the community setting.

One method of reducing the infection rate associated with catheterization has been to use silver alloy coated catheters. This has been shown in clinical trials to reduce the infection rate by up to 40% with short term catheterization (up to 14 days), but the effect in longer term catheterization is equivocal. Silver alloy catheters generally cost 3 to 4 times more than conventional catheters and thus place a considerable additional cost burden on the healthcare system.

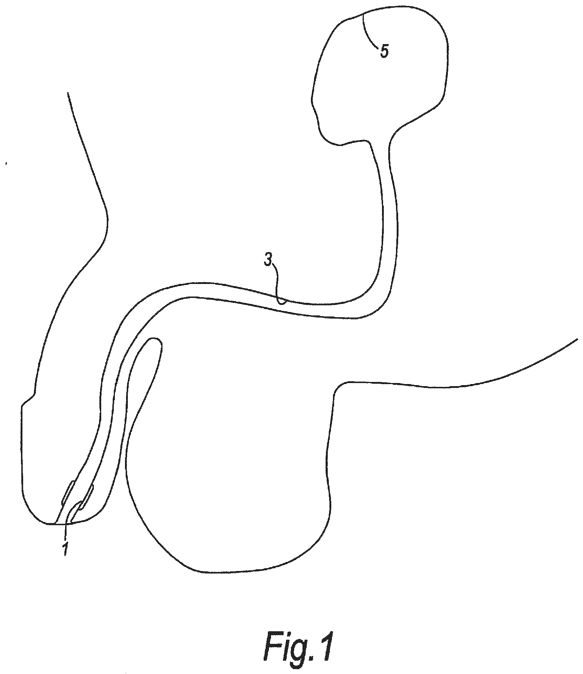

Referring to Figure 1, microbiological studies have demonstrated that the distal part 1 of the urethra 3 (the 1-2 cm furthest from the bladder 5) is colonized with bacteria. With conventional catheters these bacteria are simply pushed along the urethra into the bladder during the catheterization process. With short term catheterization (up to two weeks) one of the main mechanisms of catheter induced infection is thought to be inoculation of the catheter tip with bacteria as it is pushed along the urethra. These bacteria then sit in the tip of the catheter when in the bladder and can go on to cause a urinary tract infection.

Intermittent catheterization, where a catheter is temporarily passed into the bladder and removed once it has drained, has also been shown to significantly reduce catheter associated urinary tract infection. This infection rate can be further reduced by around 30% with the use of a urethral introducer (Bennett et al. Journal of Urology, 158, 519-521, 1997). The introducer described by Bennett et al bypassed the distal 15mm of the urethra, meaning that the catheter does not come into contact with the colonized distal urethra. One problem of this method is that the introducer simply pushes the distal urethral bacteria further along the urethra. In addition this device can only be used with intermittent rather than indwelling catheters as it can only be removed when the catheter is removed.
The commonly-assigned patent EP 1315444 discloses an eversible double-walled body cavity liner.

Also, US Patent No. 5236423 describes the eversion of a liner into a body cavity and the subsequent insertion of an endoscope within the liner. Generally such liners comprise a single walled tube of flexible material. Opposite ends of the liner are secured in position and fluid is pumped between these opposite ends to cause the liner to evert.

US Patent No. 5458573 discloses a toposcopic dilatation catheter system utilizing a dilatation balloon in combination with an everting tube in a miniature catheter of a scale sufficiently small to negotiate a blood vessel for therapeutic as well as diagnostic purposes. The catheter system utilizes a primary catheter shaft provided with multiple lumen or passageways extending the length thereof. The dilatation balloon lumen provides access to a balloon carried at the distal end of the primary catheter shaft. The dilatation balloon may be expanded as required to aid in advancing the catheter system to the site requiring therapy. A secondary catheter tube is coaxially carried within the primary catheter shaft. The leading end of the secondary catheter tube includes an everting tube which everts from the leading end of the primary catheter shaft. The everting tube advances under fluid pressure in advance to the leading end of the primary catheter shaft.

EP0247559 discloses a catheter with device to facilitate insertion based on the principle of a tube which opens out when subjected to hydraulic or pneumatic pressure. The catheter is located entirely inside a flexible thin-walled tube structure closed at the end distant from the patient. The tube structure is inserted from the end close to the patient, into the space between the catheter and the portion which is not tucked inside.

### Brief Summary of the Invention

An object of one embodiment of the invention is to provide an improved arrangement for performing catheterisation.

According to a first aspect of the present invention, there is provided a lining device as defined in claim 1.

Optional features of the lining device are defined in the dependent claims.

The cavity may be a body cavity of an inanimate object, including an excised or artificial human or animal body part. The cavity may also be a cavity of the human or animal body, such as the urethra or colon.

In the embodiment to be described in more detail below, the chamber has a closed end for facilitating the application of fluid pressure.

Preferably, the liner is attached along its length to the chamber. In the embodiment, the eversion of the liner causes the liner to extend longitudinally in the cavity.

Only a portion of the cavity may be lined by the liner. For example, when the cavity is a urethra, only the distal part of the urethra (the 1-2 centimetres furthest from the bladder) is lined by the liner, as this is the part of the urethra that is found to be colonised with bacteria. In the embodiment, after eversion of the chamber and liner into the cavity, fluid pressure is no longer applied to the chamber, which therefore deflates, facilitating the insertion of the instrument into the cavity through the liner. The instrument may be a urinary catheter. In an embodiment the liner remains in its everted position at the distal part of the urethra and the catheter slides through the liner and up the urethra to the bladder. Highly advantageously, the catheter never comes into contact with the distal part of the urethra that is colonised with bacteria and therefore may significantly reduce the likelihood of a urinary tract infection being caused by catheterisation.

The lining device may include respective liners (including the aforesaid liner) positioned at radially spaced intervals around the chamber when the chamber is everted. This advantageously allows more than one part to be inserted into the cavity (one part being inserted through each liner). This may be particularly advantageous in embodiments where the liners used for cables to be installed in underground conduits.

### Brief Description of the Drawings

For a better understanding of the present invention, an embodiment will now be described by way of example, with reference to the accompanying drawings, in which:
Figure 1 shows a diagrammatic view of the urethra and bladder of a male human body;
Figure 2 is a side elevational view of an everted lining device in accordance with an embodiment of the invention;
Figure 2A is a cross-section taken along the line A-A of Figure 2;
Figure 3 is a perspective view of the everted lining device of Figure 2;
Figure 4 is a perspective view of the everted lining device of Figure 2 showing a catheter inserted through the liner of the lining device;
Figure 4A is a cross-section taken along the line B-B of Figure 4;
Figure 5 shows a side elevational view of the holding container in which the lining device is stored prior to eversion;
Figure 6 shows a perspective view of the holding container of Figure 5 to which is attached a syringe for supplying the fluid for everting the lining device;
Figure 7 shows the everted lining device at the distal end of the urethra of Figure 1;
Figure 8 shows the urethra of Figure 7 after the catheter has been installed through the liner of the lining device;
Figure 9 shows a perspective view of the catheter, showing the removal of the lining device therefrom; and
Figure 10 shows the urethra after removal of the lining device.
In the drawings like elements are generally designated with the same reference sign.

### Detailed Description of Embodiment of the Invention

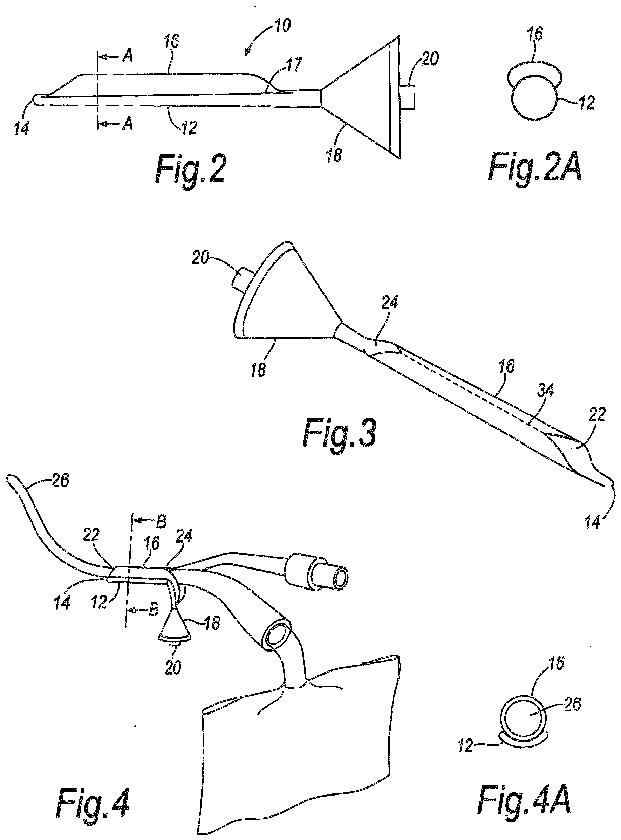

Referring initially to Figures 2 and 3, a lining device is shown in its everted state generally at 10. The lining device 10 comprises a chamber 12 that is closed at one end 14. The chamber 12 extends longitudinally in its everted state. A liner 16 is connected along its length to the chamber 12, for example by welding or any other suitable form of connection at 17.

At the opposite end of the chamber 12 to the closed end 14 a holding container 18 is attached. The holding container 18 is a hollow frusto-conical rigid part having a generally planar base (not shown) at its wider end. A fluid pipe 20 allows fluid to pass into and out of the holding container 18 through an aperture in the base.

The chamber 12 is formed from flexible material such as latex or polyurethane. The liner 16 may be formed from the same flexible material as the chamber 12. The liner 16 may be integrally formed with the chamber 12 or may be formed separately and subsequently attached thereto.

When fluid pressure is applied to the chamber 12 it is generally cylindrical and has a generally circular cross-section as shown in Figure 2A. The cross-section may not be strictly circular. The cross-section is solid - the chamber does not include any voids within the area defined by the chamber when everted. When fluid pressure is not applied to the chamber 12, the chamber may deflate and collapse upon itself.

As shown most clearly in Figure 3, the liner 16 is open at its first end 22, proximate the closed end 14 of the chamber 12, and the liner 16 is also open at its second end 24, proximate the holding container 18. The liner 16 forms an open-ended elongate tube along which a part, such as a catheter or other medical instrument can pass.

Figures 4 and 4A show a catheter 26 passing through the liner 16.

As indicated above, Figures 2,3 and 4 show the lining device 10 in its everted state. The lining device 10 may be assembled by attaching the tubular lining 16 to the chamber 12 (when in the everted state as shown in Figures 2 and 3) and welding the liner 16 by a single longitudinal weld or adhesive 17 to the chamber 12, leaving the first and second ends 22,24 of the liner 16 open. Alternatively, the liner 16 may be formed such that it fits around the circumference of the chamber 12 and slides over the chamber 12. The liner 16 is then fixed to the chamber 12 by a weld or adhesive.

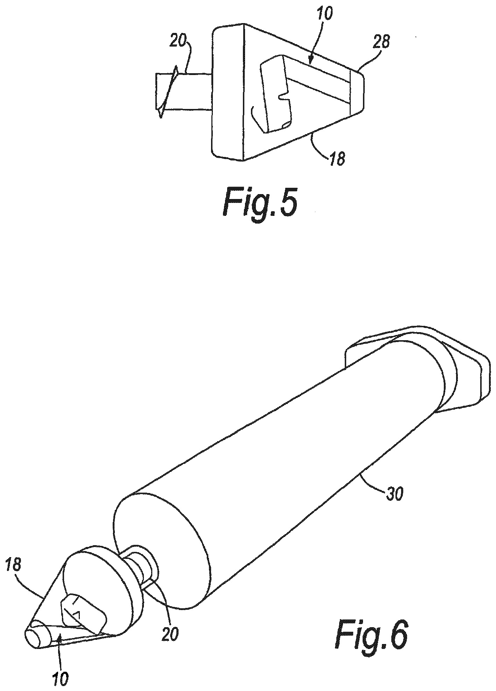

Subsequently, the open end of the chamber 12 (opposite to the closed end 14) is connected to the holding chamber 18 and a vacuum is applied, causing the chamber 12 and the attached liner 16 to be inverted into the holding container 18. An end cap 28 (Figure 5) can then be fitted and the tube 20 can be sealed (after the optional step of sterilisation). The device can then be despatched. The lining device 10 will be distributed and stored in a non-everted (inverted) state within the holding container 18, as shown in Figures 5 and 6. The lining device 10 in the holding container 18 is isolated and protected from contamination.

In Figures 5 and 6 the lining device 10 is shown in its collapsed non-everted state within the holding chamber 18. When it is desired to deploy the lining device in a cavity, such as the urethra of a patient, a syringe 30 or other suitable fluid pressure applying device is attached to the pipe 20, as shown in Figure 6. After removal of the end cap 28, the syringe 30 can be operated to apply fluid pressure to the chamber 12. The fluid applied may be air or any other fluid (liquid or gas).

For example, if it is wished to deploy the lining device 10 in the urethra of a male human patient, the holding container 18 is inserted into the opening at the distal end of the urethra at the tip of the patient's penis. The conical tip of the holding container 18 enters the first 5-10mm of the urethra 3.

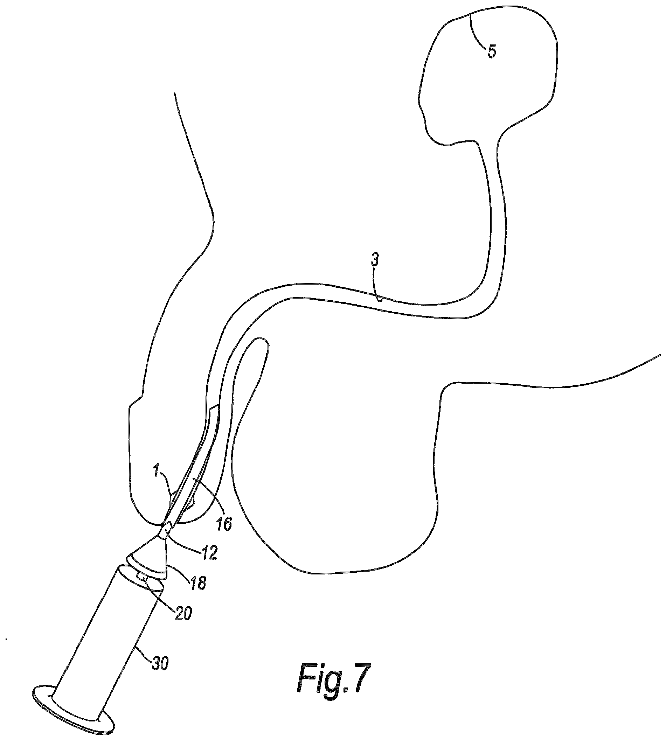

As the syringe 30 is operated, fluid passes into the holding container 18 and causes the chamber 12 to gradually evert into the urethra 3. Figure 7 shows the chamber 12 fully everted in the urethra 3. As the chamber 12 is everted into the urethra 3, due to the attachment of the liner 16 thereto, the liner 16 is drawn into the urethra and extends longitudinally along the urethra.

After eversion of the lining device 10 into the distal end of the urethra 3, the syringe 30 may be detached from the holding container 18. Removal of the syringe 30 from the pipe 20 of the holding container 18 allows the fluid in the chamber 12 to escape and the chamber 12 to completely deflate.

When fluid pressure is no longer applied to the chamber 12, the chamber 12 collapses so that it lies flat against the surface of the urethra 3 and has a significantly reduced volume (it is now generally planar in cross-section rather than circular).

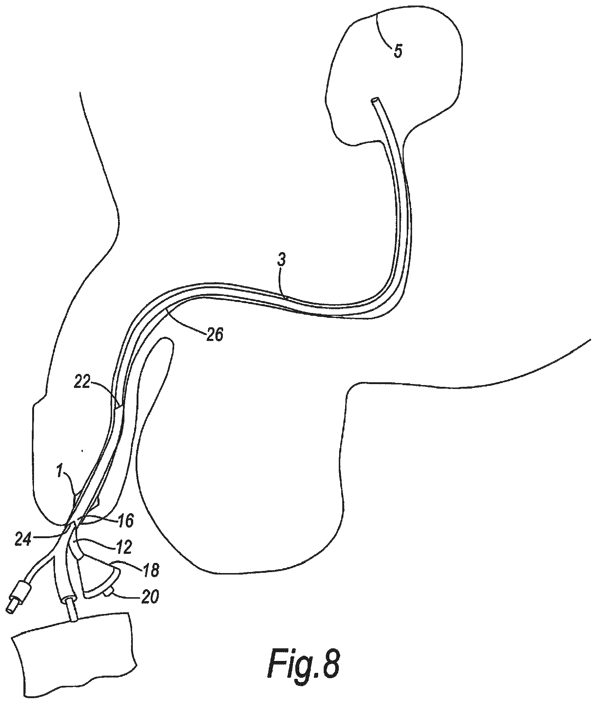

In order to catheterise the patient, as shown in Figure 8, the tip of the catheter 26 is passed into the second opening 24 of the liner 16. This causes the liner 16 to open up to accommodate the catheter 26. The catheter 26 passes along the tube formed by the liner 16. The catheter 26 slides through the liner 16 and exits through the opening at the first end 22 and then passes up the urethra 3 until it reaches the patient's bladder 5.

The part 1 of the distal urethra 3 (the 1-2 centimetres furthest from the bladder 5), that is colonised with bacteria, does not contact the catheter 26. The catheter 26 is shielded from the part 1 of the urethra by the liner 16. The catheter 26 therefore does not carry bacteria from the part 1 up the urethra 3 to the bladder 5, and therefore the risk of the urinary tract infection is significantly reduced.

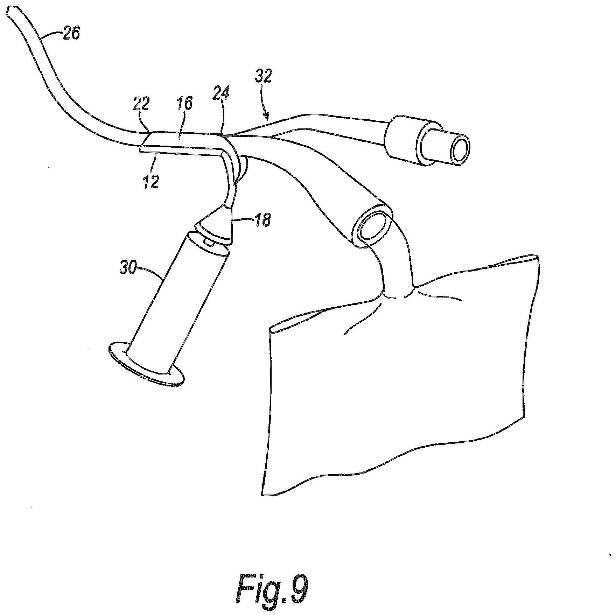

After the catheterisation is complete, as shown in Figure 8, the lining device 10 can then be removed from the urethra 3, leaving the catheter 26 in position. As shown in Figure 9, the lining device 10 may be removed by pulling it down the catheter 22 to the channel junction 32 of the catheter. The catheter 22 is widest at the junction 32, and this causes the liner 12 to split. A line of weakness 34 (Figure 3) may be provided in the liner 12 to facilitate this splitting. After the liner 16 has split along its entire length, the lining device (the liner 16 and the chamber 12) may be removed from the patient and discarded.

Optionally, the syringe 13 may be attached (if it was detached) to the holding container 18 and used to apply negative pressure (suction) via the pipe 20 to the holding container 18. This will cause the lining device 10 to invert into the holding container 18. The end cap 28 may then be replaced and the pipe 20 may be sealed after detachment from the syringe 30. The container 18, containing the lining device 10 (which is contaminated with bacteria from the part 1 of the urethra) can then be handled with reduced risk of the bacteria being spread and can subsequently be discarded.

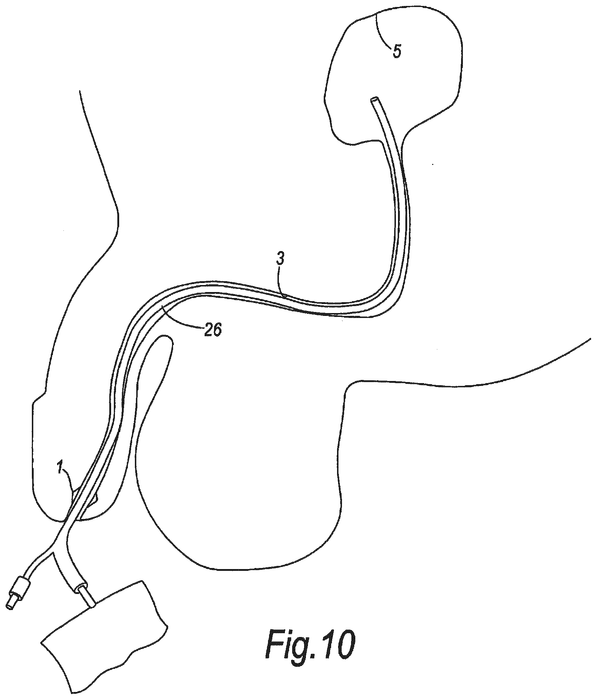

As shown in Figure 10, the catheter 26 may remain in place in the urethra 3 after removal of the lining device 1.

It should be understood that the embodiment reduces or eliminates the potential for urinary tract infection resulting in catheterisation. The embodiment provides a temporary protective lining between the inner wall of the urethra 3 and the advancing catheter 22. This prevents the bacteria at the distal part 1 of the urethra 3 from being transported further along the urethra 3 and into the bladder 5 by the action of insertion of the catheter 22 into the urethra.

A significant advantage of this embodiment of the invention is that the lining device 10 is everted into the urethra 3. The eversion causes the everting surface of the lining device 10 to lay onto the surface of the urethra 3 as eversion takes place, thus covering any bacteria 1 in the urethra, rather than pushing the bacteria further along the urethra, towards the bladder 5.

The lining device 10 may include phosphorycholine. For example, the liner 16 may be coated on its exterior with phosphorycholine-containing polymers. Such a coating may advantageously reduce friction between the liner 16 and the urethra 3 (or other body cavity). The interior surface of the liner 16 may also be coated with a phosphorycholine-containing polymer to reduce friction between the liner 16 and the catheter 22 (or any other instrument). Suitable phosphorycholine materials are available from Vertellus of Basingstoke, United Kingdom.

Phosphorycholine is zwitterionic: it has a positive and negative charge on the same molecule but is electrochemically neutral overall. Phosphorycholine therefore has high polarity and consequently a natural affinity to water. A substrate that includes phosphorycholine material is surrounded by molecular layers of water, which effectively forms a barrier over the substrate. The barrier provides a "non-stick" biological surface that resists protein and cell adhesion.

Other friction reducing polymers may be used.

The apparatus described in the embodiment may be used in many applications, not just those mentioned in relation to the embodiments. The body cavity may be a colon. In particular, the apparatus of the embodiment may be used in body cavities that are not body cavities of the human or animal body. The cavity may be the body cavity of an inanimate object, including an excised or artificial human or animal body part.

The apparatus described in the embodiment may also be of any length or diameter. The chamber 12 and/or liner may have a length sufficient to line the entire urethra 3 (or colon).

## Claims

1. A lining device for lining a body cavity and for facilitating the insertion of an instrument into the cavity (3), the lining device comprising an eversible chamber (12), the arrangement being such that when fluid pressure is applied to the chamber (12) it causes the chamber to evert and advance into the cavity (3), **characterised in that** the lining device comprises a liner (16) connected to the chamber (12) such that the chamber (12) is operable to carry the liner (16) into the cavity (3) so that the liner (16) provides a passage on the exterior of the chamber (12) within the cavity (3), the reduction of fluid pressure applied to the chamber (12) causing deflation of the chamber (12), and the liner (16) being expandable in response to insertion of the instrument to accommodate the instrument in the passage such that the passage has an external diameter equal to the internal diameter of the cavity (3).

2. The lining device of claim 1, wherein the chamber (12) has a closed end for facilitating the application of the fluid pressure thereto.

3. The lining device of claim 1 or 2, wherein the liner (16) is attached along its length to the chamber (12).

4. The lining device of claim 1,2 or 3 wherein the liner (16) is arranged to line only a portion of the cavity (3).

5. The lining device of any one of claims 1 to 4, wherein the liner (16) includes a line of weakness (34) to facilitate splitting of the linear (16) to allow removal of the liner (16) from the instrument (26) whilst the instrument (26) remains in the cavity (3).

6. The lining device of any one of claims 1 to 5, wherein the liner (16) is configured to line the cavity (3) at one end region thereof and is arranged such that the instrument is slidable along the liner (16) to allow the instrument to be inserted into the cavity (3) such that the instrument reaches a region of the cavity (3) other than the end region.

7. The lining device of any one of claims 1 to 6, wherein the instrument comprises a catheter and the cavity comprises a urethra.

8. The lining device of any one of claims 1 to 7, including respective liners positioned at radially spaced intervals around the chamber (12) when the chamber (12) is everted.

9. The lining device of any one of claims 1 to 8, wherein the eversible chamber (12) has a solid cross-section and/or is generally cylindrical.

## Patentansprüche

1. Auskleidungsvorrichtung zum Auskleiden einer Körperhöhle und zum Erleichtern der Einführung eines Instruments in die Körperhöhle (3), wobei die Auskleidungsvorrichtung eine umstülpbare Kammer (12) umfasst, wobei die Anordnung so ist, dass beim Beaufschlagen der Kammer (12) mit Flüssigkeitsdruck verursacht wird, dass sich die Kammer umstülpt und in die Höhle (3) vorschiebt, **dadurch gekennzeichnet, dass** die Auskleidungsvorrichtung eine Auskleidung (16) umfasst, die so mit der Kammer (12) verbunden ist, dass die Kammer (12) dazu dient, die Auskleidung (16) in die Höhle (3) zu tragen, so dass die Auskleidung (16) einen Durchgang auf der Außenseite der Kammer (12) innerhalb der Höhle (3) bereitstellt, wobei die Verringerung des auf die Kammer (12) aufgebrachten Flüssigkeitsdrucks Entleeren der Kammer (12) verursacht, und wobei die Auskleidung (16) als Reaktion auf die Einführung des Instruments expandierbar ist, um das Instrument im Durchgang aufzunehmen, so dass der Durchgang einen Außendurchmesser aufweist, der dem Innendurchmesser der Höhle (3) gleicht.

2. Auskleidungsvorrichtung nach Anspruch 1, wobei die Kammer (12) ein geschlossenes Ende zur Erleichterung des Aufbringens von Flüssigkeitsdruck darauf aufweist.

3. Auskleidungsvorrichtung nach Anspruch 1 oder 2, wobei die Auskleidung (16) entlang ihrer Länge an der Kammer (12) befestigt ist.

4. Auskleidungsvorrichtung nach Anspruch 1, 2 oder 3, wobei die Auskleidung (16) dazu angeordnet ist, nur einen Teil der Höhle (3) auszukleiden.

5. Auskleidungsvorrichtung nach einem der Ansprüche 1 bis 4, wobei die Auskleidung (16) eine Schwächungslinie (34) zur Erleichterung des Trennens der Auskleidung (16) aufweist, um die Entfernung der Auskleidung (16) von dem Instrument (26) zu erlauben, während das Instrument (26) in der Höhle (3) verbleibt.

6. Auskleidungsvorrichtung nach einem der Ansprüche 1 bis 5, wobei die Auskleidung (16) dazu ausgelegt ist, die Höhle (3) an einem Endbereich davon auszukleiden und so angeordnet ist, dass das Instrument entlang der Auskleidung (16) gleiten kann, damit das Instrument in die Höhle (3) eingeführt werden kann, so dass das Instrument einen anderen Bereich der Höhle (3) als den Endbereich erreicht.

7. Auskleidungsvorrichtung nach einem der Ansprüche 1 bis 6, wobei das Instrument einen Katheter umfasst und die Höhle eine Harnröhre umfasst.

8. Auskleidungsvorrichtung nach einem der Ansprüche 1 bis 7, umfassend jeweilige Auskleidungen, die in radial beabstandeten Intervallen um die Kammer (12) angeordnet sind, wenn die Kammer (12) umgestülpt ist.

9. Auskleidungsvorrichtung nach einem der Ansprüche 1 bis 8, wobei die umstülpbare Kammer (12) einen festen Querschnitt aufweist und/oder allgemein zylinderförmig ist.

## Revendications

1. Dispositif de doublure pour doubler une cavité corporelle et pour faciliter l'introduction d'un instrument dans la cavité (3), le dispositif de doublure comprenant une chambre pouvant être éversée (12), l'agencement étant tel que, lorsqu'une pression fluidique est appliquée à la chambre (12), elle amène la chambre à s'éverser et à avancer dans la cavité (3), **caractérisé en ce que** le dispositif de doublure comprend une doublure (16) reliée à la chambre (12) de sorte que la chambre (12) puisse fonctionner pour transporter la doublure (16) dans la cavité (3) de sorte que la doublure (16) forme un passage sur l'extérieur de la chambre (12) à l'intérieur de la cavité (3), la réduction de la pression fluidique appliquée à la chambre (12) provoquant le dégonflement de la chambre (12), et la doublure (16) étant expansible en réponse à l'introduction de l'instrument pour accueillir l'instrument dans le passage de sorte que le passage ait un diamètre externe égal au diamètre interne de la cavité (3).

2. Dispositif de doublure selon la revendication 1, dans lequel la chambre (12) possède une extrémité fermée pour faciliter l'application de la pression fluidique à celle-ci.

3. Dispositif de doublure selon la revendication 1 ou 2, dans lequel la doublure (16) est fixée sur sa longueur à la chambre (12).

4. Dispositif de doublure selon la revendication 1, 2 ou 3 dans lequel la doublure (16) est conçue pour doubler uniquement une partie de la cavité (3).

5. Dispositif de doublure selon l'une quelconque des revendications 1 à 4, dans lequel la doublure (16) comprend une ligne de faiblesse (34) pour faciliter la division de la doublure (16) pour permettre le retrait de la doublure (16) de l'instrument (26) pendant que l'instrument (26) reste dans la cavité (3).

6. Dispositif de doublure selon l'une quelconque des revendications 1 à 5, dans lequel la doublure (16) est conçue pour doubler la cavité (3) au niveau d'une région d'extrémité de celle-ci et est conçue de sorte que l'instrument puisse coulisser le long de la doublure (16) pour permettre que l'instrument soit introduit dans la cavité (3) de sorte que l'instrument atteigne une région de la cavité (3) différente de la région d'extrémité.

7. Dispositif de doublure selon l'une quelconque des revendications 1 à 6, dans lequel l'instrument comprend un cathéter et la cavité comprend un urètre.

8. Dispositif de doublure selon l'une quelconque des revendications 1 à 7, comprenant des doublures respectives positionnées au niveau d'intervalles radialement espacés autour de la chambre (12) lorsque la chambre (12) est éversée.

9. Dispositif de doublure selon l'une quelconque des revendications 1 à 8, dans lequel la chambre pouvant être éversée (12) possède une coupe transversale pleine et/ou est généralement cylindrique.
